# EUROPEAN PATENT APPLICATION

(11) **EP 3 689 334 A1**
(43) Date of publication of application: **05.08.2020**
(21) Application number: 20151213.4
(22) Date of filing: 11.03.2015
(51) Int. Cl.: A61K 9/08, A61P 25/16, A61P 5/06, A61P 25/14, A61P 25/26, A61P 25/28, A61K 31/198, A61K 31/4045, A61K 31/428, A61K 31/506

(54) **COMPOSITION COMPRISING A DOPAMINE AGONIST AND AN L-DOPA DERIVATIVE FOR TREATING PARKINSON'S DISEASE**

(30) Priority: 11.03.2014 EP 14158948
(62) Divisional of application: 15712819.0
(71) Applicant: Teva Pharmaceuticals International GmbH, 8645 Jona (CH)
(72) Inventor: ALKEN, Rudolf-Giesbert, S-233 35 Svedala (SE); SCHNEIDER, Frank, 12437 Berlin (DE)
(74) Representative: D Young & Co LLP

(57) **Abstract**

Disclosed is a pharmaceutical composition comprising i) a dopamine agonist and ii) a L-DOPA derivative in combination in form of a liquid preparation. The pharmaceutical composition is used for the treatment of Parkinson's disease, restless leg syndrome, dystonia, for inhibiting prolactin secretion, for stimulating the release of growth hormones, for the treatment of neurological symptoms of chronic manganese intoxication, amyotrophic lateral sclerosis, and multiple system atrophy.

## Description

Disclosed herein are new compositions and their application as pharmaceuticals for the treatment of disorders. Methods of modulating neurotransmitter levels in a subject are also provided for the treatment of disorders such as Parkinson's disease, restless leg syndrome, dystonia, for inhibiting prolactin secretion, for stimulating the release of growth hormones, for the treatment of neurological symptoms of chronic manganese intoxication, amyotrophic lateral scleroses, and multiple system atrophy.

Levodopa (L-3-(3,4-dihydroxyphenyl)-alanine; (-)-3,4-dihydroxyphenylalanine; (-)-dopa; (2S)-2-amino-3-(3,4-dihydroxyphenyl)propanoic acid; (S)-3,4-dihydroxyphenylalanine; 3,4-dihydroxy-L-phenylalanine; 3,4-dihydroxyphenyl-L-alanine; 3,4-dihydroxyphenylalanine; 3-(3,4-dihydroxyphenyl)-L-alanine; 3-hydroxy-L-tyrosine; 3-hydroxytyrosine; alphadopa; bendopa; brocadopa; cidandopa; DA; DOPA; deadopa; dihydroxy-L-phenylalanine; dihydroxyphenylalanine; Dopaflex®; Dopaidan®; Dopal®; Dopalina®; Dopar®; Doparkine®; Doparl®; Dopasol®; Dopaston®; Dopaston SE®; Dopastone®; Dopastral®; Dopicar®; Doprin®; Eldopal®; Eldopar®; CAS # 59-92-7) is a neurotransmitter modulator. In the body levodopa is converted to dopamine by the action of the enzyme L-aromatic-amino-acid decarboxylase.

Parkinson's disease is a neurodegenerative disease with a slow progressive course characterized by different symptoms and signs that may be present or develop during the progression of disease. Core symptoms are bradykinesia and at least one of the following, namely resting tremor, muscular rigidity and postural reflex impairment. Other symptoms that may occur during the disease progression are autonomic disturbances, sleep disturbances, disturbances in the sense of smell or sense of temperature as well as depressive symptoms and cognitive dysfunctions.

The improvement of the impaired dopaminergic neurotransmission by administration of L-DOPA is the backbone of the current pharmacotherapy. Patients with advanced Parkinson's disease require higher doses of dopaminergics which is limited by motor complications, like fluctuations and involuntarily movements (described as levodopa induced dyskinesia, LIDs). Fluctuations might be due to the shorter striatal persistence (half life) of dopamine especially in advanced Parkinson's disease patients. A clinical established approach to prolong striatal dopamine persistence is the co-administration of MAO-B inhibitors which block the main metabolic breakdown route of dopamine. The induction of LIDs is associated in many patients with higher CNS dopamine levels generated by large L-DOPA doses.

Apomorphine is a derivative of morphine but does not share the major characteristics of morphine such as soothing the pain, acting euphoric or loss of effectiveness. Apomorphine is a non-selective dopamine agonist which activates both D1-like and D2-like receptors and is currently used in the treatment of Parkinson's disease. Apomorphine is used for the treatment of Parkinson's patients with insufficient control of motor fluctuations despite individually optimized L-DOPA (L-DOPA + DOPA decarboxylase inhibitor) and /or dopamine agonist therapy in the form of a subcutaneous injection or as a permanent infusion.

Three solutions currently known using apomorphine for intermittent subcutaneous injections are marketed under different trademarks (APO-go® PFS, APO-go® ampoules and Apokyn®). But these formulations cause injection site reactions such as nodulation and the formation of discoloured spots due to their low pH (range 3.0-4.0).

WO 2013007381 describes a new formulation with apomorphine as the active substance wherein the pH is greater than 4 in order to prevent any reactions at the site of injection.

The current therapy of late Parkinson's disease with subcutaneous infusion of apomorphine allows a dose reduction but requires the additional administration of oral dopaminergics to control motor fluctuations. A daily dose of infused apomorphine at 72.00 ± 21.38 mg reduced the doses of oral L-DOPA from 989.4 ± 420.1 mg/day to 663.8 ± 403.2 mg/day (Ruiz et al., Mov Disord, 2008, 23(8): 1130-1136). Further a reduction of 55% of L-DOPA was reported using a mean daily apomorphine dose of 75.2 mg (Katzenschlager et al., 2005, Mov Disord, 20(2): 151-157).

To compare the effects of changes in antiparkinsonian medications, the daily dopaminergic treatment can be calculated as levodopa equivalent dose (LED) using the method decribed by Deuschl et al. (N Engl J Med 2006; 355: 898). A 10 mg daily dose of apomorphine is equivalent to a standard levodopa dose of 100 mg (LED = 100 mg). A 100 mg dose of levodopa accounts for 100 mg LED.

In addition, levodopa is administered in combination with active additives in pharmaceuticals. Combinations of levodopa are used with peripheral decarboxylase inhibitors, with inhibitors of the enzyme catechol-O-methyltransferase (COMT), with inhibitors of the enzyme monoamine oxidase (MAO) and with dopamine β-hydroxylase inhibitors.

In this connection, the decarboxylase inhibitors used are, for example: D,L-serine 2-(2,3,4-trihydroxybenzyl) hydrazide (benserazide), (-)-L-α-hydrazino-3,4-dihydroxy-α-methylhydrocinnamic acid (carbidopa), L-serine-2-(2,3,4-trihydroxybenzyl) hydrazide, glycine-2-(2,3,4-trihydroxybenzyl)hydrazide and L-tyrosine-2-(2,3,4-trihydroxybenzyl)hydrazide. Examples of combination preparations of levodopa and decarboxylase inhibitors include, among others: Madopar® (levodopa and benserazide hydrochloride) as well as Nacom® (levodopa and carbidopa).

In Duodopa®-pumps a solution of L-DOPA/Carbidopa is used for continuous administration into the small intestine of Parkinson patients. The treatment with Duodopa is used in patients with advanced Parkinson's disease who do not have satisfactory control of severe, disabling motor symptoms with available combinations of medications for Parkinson's disease. The treatment requires a surgery to insert a tube directly into the upper small intestine. Before surgery, a temporary tube through the nose into the small intestine will be used to control response and adjust the dose. Most patients on Duodopa don't need additional oral L-DOPA administration. But the limitations of the gastric transport barrier remain.

Further, at high doses of Duodopa, dyskinesia is present, which is possibly due to the formation of norepinephrine an unavoidable metabolite of L-DOPA and alpha receptor agonist. Alpha blockers have been shown to reduce L-DOPA induced dyskinesia (LIDs) (Lewitt, 2012, Transl. Neurodegener., 1(1): 4). Currently there are different pharmacological means under development to treat existing LIDs.

Many attempts have been made to study L-DOPA induced dyskinesia. In order to study the effects of co-administration of L-DOPA and dopamine agonists like apomorphine or ropinirole, both drugs have been either administered on different pathway or orally together. Hill et al. (Michael P Hill et al.: "Novel antiepileptic drug levetiracetam decreases dyskinesia elicited by L-dopa and ropinirole in the MPTP-lesioned marmoset", Movement disorders: official journal of the Movement Disorder Society, 1 November 2003, pages 1301-1305) describe the oral administration of a mixture of L-DOPA and ropinirole dissolved in apple juice to mice. Baas et al. (BAAS H et al.: "Pharmacodynamics of levodopa coadministered with apomorphine in Parkinsonian patients with end-of-dose motor fluctuations", CLINICAL PRESS, AUCKLAND, NZ, vol. 14, no. 6, 1 January 1996, pages 365-374). Herein co-medication with DOPA carboxylase inhibitors is suggested in order to mage dyskinesia in parkinsonian patients. NEUROPHARMACOLOGY, RAVEN PRESS, NEW YORK, NY, US, vol. 21, no. 2, 1 March 1998, pages 86-92) describe the co-medication of subcutaneous apomorphine administration in combination with oral single dose applications of L-DOPA/benserazide in patients. Giron et al. propose methods of managing L-Dopa induced dyskinesias (Giron L T et al.: "METHODS OF MANAGING LEVODOPA-INDUCED DYSKINESIAS", DRUG SAFETY, ADIS PRESS, AUCKLAND, NZ, vol. 14, no. 6, 1 January 1996, pages 365-374). Herein co-medication with DOPA carboxylase inhibitors is suggested in order to manage dyskinesia in parkinsonian patients.

None of the named publications suggest the non-oral administration of a liquid combination of L-DOPA or its derivatives together with a dopamine agonist. No pharmaceutical preparation is disclosed that comprises L-DOPA or its derivatives together with a dopamine agonist in combination to be administered non-orally by injection or infusion or via gastric or enteral application.

None of the named publications suggest the non-oral administration of a liquid combination of L-DOPA or its derivatives together with a dopamine agonist. No pharmaceutical preparation is disclosed that comprises L-DOPA or its derivatives together with a dopamine agonist in combination to be administered non-orally by injection or infusion or via gastric or enteral application.

α,β,β-D3-L-DOPA exhibited higher longer-lasting striatal dopamine levels than L-DOPA. Correspondingly to the increased availability of dopamine in the striatum, α,β,β-D3-L-DOPA showed improved motor activity compared to L-DOPA in several Parkinson models (Malmlof et al., Exp Neurol, 2008, 538-542; Malmlof et al., Exp Neurol, 2010, 225: 408-415). The equi-effective dose of α,β,β-D3-L-DOPA compared to L-DOPA was about 60%. The observed longer striatal persistence of dopamine allowed the assumption that fluctuations might be reduced as well.

S/S-2-amino-2,3-dideutero-3-(3,4-dihydroxyphenyl) propionic acid (α,β-D2-L-DOPA) and L-2-Amino-2,3,3-trideutero-3-(3,4-dihydroxyphenyl) propionic acid (α,β,β-D3-L-DOPA) were shown to increase and prolong the output of striatal dopamine significantly more than L-DOPA (WO 2004056724 and WO 2007093450).

The highest striatal dopamine concentrations were found after administration of α,β-D2-L-DOPA. Those dopamine levels were even higher than those after the administration of the triple-deuterated α,β,β-D3-L-DOPA which included the same deuterated positions as the double deuterated L-DOPA.

At the equi-effective dose (same striatal dopamine levels and same motor effect as L-DOPA), α,β,β-D3-L-DOPA caused significant less dyskinesia than L-DOPA (Malmlof et al., Exp Neurol, 2010, 225: 408-415).

The problem to be solved according to the invention is to overcome the drawbacks described in the literature and to provide a new pharmaceutical composition according to the main claim for the treatment of Parkinson's disease.

Although resting tremor is the most identifiable sign of Parkinson's disease, its underlying basis appears to be the most complex of the cardinal signs. Although levodopa is clearly effective for resting tremor, several agents have shown efficacy that appears to be superior or additive to that of levodopa in-cluding anticholinergics, clozapine, pramipexole, and budipine.

The evaluation of the efficacy and safety of adjuvant treatment to levodopa therapy in Parkinson's disease patients with motor complications revealed no significant difference between dopamine agonists and placebo.

In a MPTP monkey model of Parkinson's disease DP-102 (α,β,β-D3-L-DOPA) showed less tremor at more Good ON as compared to the same dose of L-DOPA. Details of the MPTP monkey model used are described below.

The inventors have found that the local tolerance of the pharmaceutical composition containing deuterated L-DOPA derivatives according to the invention is better as compared to un-deuterated L-DOPA at equi-effective dose.

The local tolerance can be enhanced by addition of cytoprotective compounds like N-acetylcysteine, cysteine or alpha lipoic acid.

### Deuterium Kinetic Isotope Effect

In order to eliminate foreign substances such as therapeutic agents, the animal body expresses various enzymes, such as the cytochrome P₄₅₀ enzymes (CYPs), esterases, proteases, reductases, dehydrogenases, and monoamine oxidases, to react with and convert these foreign substances to more polar intermediates or metabolites for renal excretion. Such metabolic reactions frequently involve the oxidation of a carbon-hydrogen (C-H) bond to either a carbon-oxygen (C-O) or a carbon-carbon (C-C) π-bond. The resultant metabolites may be stable or unstable under physiological conditions, and can have substantially different pharmacokinetic, pharmacodynamic, and acute and long-term toxicity profiles relative to the parent compounds. For most drugs, such oxidations are generally rapid and ultimately lead to administration of multiple or high daily doses.

The relationship between the activation energy and the rate of reaction may be quantified by the Arrhenius equation, k = Ae^{-Eact/RT}. The Arrhenius equation states that, at a given temperature, the rate of a chemical reaction depends exponentially on the activation energy (E_{act}).

The transition state in a reaction is a short lived state along the reaction pathway during which the original bonds have stretched to their limit. By definition, the activation energy E_{act} for a reaction is the energy required to reach the transition state of that reaction. Once the transition state is reached, the molecules can either revert to the original reactants, or form new bonds giving rise to reaction products. A catalyst facilitates a reaction process by lowering the activation energy leading to a transition state. Enzymes are examples of biological catalysts.

Carbon-hydrogen bond strength is directly proportional to the absolute value of the ground-state vibrational energy of the bond. This vibrational energy depends on the mass of the atoms that form the bond, and increases as the mass of one or both of the atoms making the bond increases. Since deuterium (D) has twice the mass of protium (¹H), a C-D bond is stronger than the corresponding C-¹H bond. If a C-¹H bond is broken during a rate-determining step in a chemical reaction (i.e. the step with the highest transition state energy), then substituting a deuterium for that protium will cause a decrease in the reaction rate. This phenomenon is known as the Deuterium Kinetic Isotope Effect (DKIE). The magnitude of the DKIE can be expressed as the ratio between the rates of a given reaction in which a C-¹H bond is broken, and the same reaction where deuterium is substituted for protium. The DKIE can range from about 1 (no isotope effect) to very large numbers, such as 50 or more. Substitution of tritium for hydrogen results in yet a stronger bond than deuterium and gives numerically larger isotope effects

Deuterium (²H or D) is a stable and non-radioactive isotope of hydrogen which has approximately twice the mass of protium (¹H), the most common isotope of hydrogen. Deuterium oxide (D₂O or "heavy water") looks and tastes like H₂O, but has different physical properties.

When pure D₂O is given to rodents, it is readily absorbed. The quantity of deuterium required to induce toxicity is extremely high. When about 0-15% of the body water has been replaced by D₂O, animals are healthy but are unable to gain weight as fast as the control (untreated) group. When about 15-20% of the body water has been replaced with D₂O, the animals become excitable. When about 20-25% of the body water has been replaced with D₂O, the animals become so excitable that they go into frequent convulsions when stimulated. Skin lesions, ulcers on the paws and muzzles, and necrosis of the tails appear. The animals also become very aggressive. When about 30% of the body water has been replaced with D₂O, the animals refuse to eat and become comatose. Their body weight drops sharply and their metabolic rates drop far below normal, with death occurring at about 30 to about 35% replacement with D₂O. The effects are reversible unless more than thirty percent of the previous body weight has been lost due to D₂O. Studies have also shown that the use of D₂O can delay the growth of cancer cells and enhance the cytotoxicity of certain antineoplastic agents.

Deuteration of pharmaceuticals to improve pharmacokinetics (PK), pharmacodynamics (PD), and toxicity profiles has been demonstrated previously with some classes of drugs. For example, the DKIE was used to decrease the hepatotoxicity of halothane, presumably by limiting the production of reactive species such as trifluoroacetyl chloride. However, this method may not be applicable to all drug classes. For example, deuterium incorporation can lead to metabolic switching. Metabolic switching occurs when xenogens, sequestered by Phase I enzymes, bind transiently and re-bind in a variety of conformations prior to the chemical reaction (e.g., oxidation). Metabolic switching is enabled by the relatively vast size of binding pockets in many Phase I enzymes and the promiscuous nature of many metabolic reactions. Metabolic switching can lead to different proportions of known metabolites as well as altogether new metabolites. This new metabolic profile may impart more or less toxicity. Such pitfalls are non-obvious and are not predictable *a priori* for any drug class.

Levodopa is a neurotransmitter modulator. The carbon-hydrogen bonds of levoidopa contain a naturally occurring distribution of hydrogen isotopes, namely ¹H or protium (about 99.9844%), ²H or deuterium (about 0.0156%), and ³H or tritium (in the range between about 0.5 and 67 tritium atoms per 10¹⁸ protium atoms). Increased levels of deuterium incorporation may produce a detectable Deuterium Kinetic Isotope Effect (DKIE) that could effect the pharmacokinetic, pharmacologic and/or toxicologic profiles of such levodopa in comparison with the compound having naturally occurring levels of deuterium.

Based on discoveries made in our laboratory, as well as considering the literature, levodopa is metabolized in humans to give dopamine which is further metabolized to form epinephrine and norepinephrine, wherein dopamine, epinephrine, and norepinephrine are each further metabolized at their N-methylene group. The current approach has the potential to prevent metabolism at this site. Other sites on the molecule may also undergo transformations leading to metabolites with as-yet-unknown pharmacology/toxicology. Limiting the production of these metabolites has the potential to decrease the danger of the administration of such drugs and may even allow increased dosage and/or increased efficacy. All of these transformations can occur through polymorphically-expressed enzymes, exacerbating interpatient variability. Further, some disorders are best treated when the subject is medicated around the clock or for an extended period of time. For all of the foregoing reasons, a medicine with a longer half-life may result in greater efficacy and cost savings. Various deuteration patterns can be used to (a) reduce or eliminate unwanted metabolites, (b) increase the half-life of the parent drug, (c) decrease the number of doses needed to achieve a desired effect, (d) decrease the amount of a dose needed to achieve a desired effect, (e) increase the formation of active metabolites, if any are formed, (f) decrease the production of deleterious metabolites in specific tissues, and/or (g) create a more effective drug and/or a safer drug for polypharmacy, whether the polypharmacy be intentional or not. The deuteration approach has the strong potential to slow the metabolism of levodopa and attenuate interpatient variability.

Novel pharmaceutical compositions, certain of which have been found to function as neurotransmitter prodrugs have been discovered, together with methods of synthesizing and using the compounds, including methods for the treatment of neurotransmitter-mediated disorders in a patient by administering the compounds.

In certain embodiments, disclosed herein is a pharmaceutical composition comprising a dopamine agonist; and a L-DOPA derivative in combination in form of a liquid preparation.

In further embodiments the pharmaceutical composition is in the form of a non-orally applicable liquid preparation.

In further embodiments the pharmaceutical composition further comprises pharmaceutically acceptable adjuvants and additives from the group of solvents, sugars or pH regulators.

In further embodiments the dopamine agonist is selected from the group comprising apomorphine, ropinirole, rotigotine, pramipexole, and piribedile.

In further embodiments the L-DOPA derivative is selected from L-DOPA, selectively and/or partially deuterated L-DOPA derivatives, as well as physiologically acceptable salts of the aforementioned L-DOPA derivatives.

In further embodiments the pharmaceutical composition contains one or more deuterated derivatives of L-DOPA as well as physiologically acceptable salts thereof.

In further embodiments the deuterated derivative of L-DOPA is selected from the group consisting of: 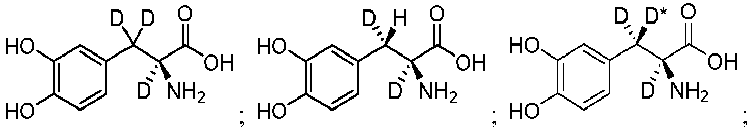 and 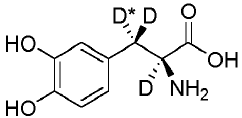 or physiological acceptable salts thereof, wherein each position designated as D or D* is enriched with deuterium.

In further embodiments each position designated as D has deuterium enrichment of no less than about 90%.

In further embodiments each position designated as D has deuterium enrichment of no less than about 96%.

In further embodiments each position designated as D has deuterium enrichment of no less than about 98%.

In further embodiments each position designated as D* has deuterium enrichment of about 80% to about 100%.

In further embodiments each position designated as D* has deuterium enrichment of about 85% to about 95%.

In further embodiments each position designated as D* has deuterium enrichment of about 88% to about 92%.

In further embodiments each position designated as D* has deuterium enrichment of about 90%.

In further embodiments the pharmaceutical composition further comprises at least one DOPA decarboxylase inhibitor.

In further embodiments the DOPA decarboxylase inhibitor is selected from the group of (-)-L-α-hydrazino-3,4-dihydroxy-α-methylhydrocinnamic acid (carbidopa), D,L-serine 2-(2,3,4-trihydroxybenzyl) hydrazide (benserazide), L-serine-2-(2,3,4-trihydroxybenzyl) hydrazide, glycine-2-(2,3,4-trihydroxybenzyl) hydrazide or L-tyrosine-2-(2,3,4-trihydroxybenzyl) hydrazide and physiological acceptable salts thereof.

In further embodiments the dopamine agonist is apomorphine and wherein the concentration of apomorphine is between 2-30 mg/ml.

In further embodiments the concentration of apomorphine is about 5 mg/ml.

In further embodiments the L-DOPA derivative is L-DOPA and wherein the concentration of L-DOPA is between 5-50 mg/ml.

In further embodiments the concentration of L-DOPA is between 10-15 mg/ml.

In further embodiments the DOPA decarboxylase inhibitor is carbidopa and wherein the concentration of carbidopa is between 0.5-10 mg/ml.

In further embodiments the concentration of carbidopa is about 2-3 mg/ml.

In further embodiments the pharmaceutical composition further comprises at least one cytoprotective compound.

In further embodiments the cytoprotective compound is selected from the group comprising N-acetylcysteine, cysteine and alpha lipoic acid

In certain embodiments, disclosed herein is a method for the preparation of the pharmaceutical composition according to at least one of the preceding claims, comprising mixing at least one dopamine agonist and at least one L-DOPA derivative in a defined ratio to each other.

In further embodiments the method further comprises a step of sterilizing the obtained mixture.

In certain embodiments, disclosed herein is the use of a pharmaceutical composition for the treatment of Parkinson's disease, restless leg syndrome, dystonia, for inhibiting prolactin secretion, for stimulating the release of growth hormones, for the treatment of neurological symptoms of chronic manganese intoxication, amyotrophic lateral scleroses, and multiple system atrophy.

In further embodiments the use is in a non-oral application form selected from infusions, injections or an application via a gastric tube or an intestine tube.

In certain embodiments, disclosed herein is a pharmaceutical composition comprising apomorphine, L-DOPA and DOPA decarboxylase inhibitor in a defined ratio to each other.

In certain embodiments, the use of a pharmaceutical composition according to the invention instead of the currently used apomorphine infusion with concomitant oral levodopa administration allows up to 40% reduction of the LED.

In certain embodiments, the pharmaceutical composition my also comprise a deuterated form of L-DOPA.

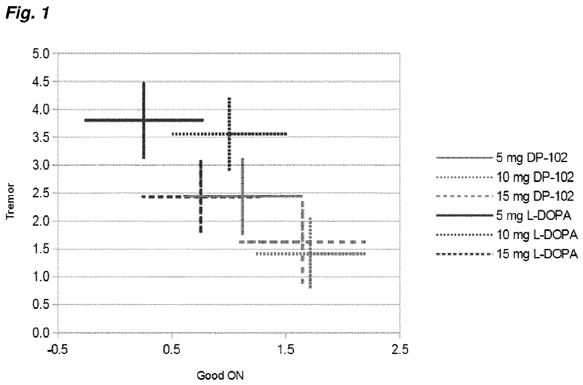

In certain embodiments, disclosed herein is a pharmaceutical composition that contains L-DOPA derivative DP-102, namely L-2-Amino-2,3,3-trideutero-3-(3,4-dihydroxyphenyl) propionic acid (α,β,β-D3-L-DOPA). In further embodiments, it is shown that triple deuterated L-DOPA is unexpectedly more suitable for the combination with dopamine agonists than L-DOPA itself. This is shown in Figure 1.

Certain compounds disclosed herein may possess useful neurotransmitter modulating activity, and may be used in the treatment or prophylaxis of a disorder in which neurotransmitter levels play an active role. Thus, certain embodiments also provide pharmaceutical compositions comprising one or more compounds disclosed herein together with a pharmaceutically acceptable carrier, as well as methods of making and using the compounds and compositions. Certain embodiments provide methods for modulating neurotransmitter activity. Other embodiments provide methods for treating a neurotransmitter-mediated disorder in a patient in need of such treatment, comprising administering to said patient a therapeutically effective amount of a compound or composition according to the present invention. Also provided is the use of certain compounds disclosed herein for use in the manufacture of a medicament for the prevention or treatment of a disorder ameliorated by the modulation of neurotransmitter levels.

The compounds as disclosed herein may also contain less prevalent isotopes for other elements, including, but not limited to, ¹³C or ¹⁴C for carbon, ³³S, ³⁴S, or ³⁶S for sulfur, ¹⁵N for nitrogen, and ¹⁷O or ¹⁸O for oxygen.

In certain embodiments, the compound disclosed herein may expose a patient to a maximum of about 0.000005% D₂O or about 0.00001% DHO, assuming that all of the C-D bonds in the compound as disclosed herein are metabolized and released as D₂O or DHO. In certain embodiments, the levels of D₂O shown to cause toxicity in animals is much greater than even the maximum limit of exposure caused by administration of the deuterium enriched compound as disclosed herein. Thus, in certain embodiments, the deuterium-enriched compound disclosed herein should not cause any additional toxicity due to the formation of D₂O or DHO upon drug metabolism.

In certain embodiments, the deuterated compounds disclosed herein maintain the beneficial aspects of the corresponding non-isotopically enriched molecules while substantially increasing the maximum tolerated dose, decreasing toxicity, increasing the half-life (T_{1/2}), lowering the maximum plasma concentration (Cₘₐₓ) of the minimum efficacious dose (MED), lowering the efficacious dose and thus decreasing the non-mechanism-related toxicity, and/or lowering the probability of drug-drug interactions.

All publications and references cited herein are expressly incorporated herein by reference in their entirety. However, with respect to any similar or identical terms found in both the incorporated publications or references and those explicitly put forth or defined in this document, then those terms definitions or meanings explicitly put forth in this document shall control in all respects.

As used herein, the terms below have the meanings indicated.

The singular forms "a," "an," and "the" may refer to plural articles unless specifically stated otherwise.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

When ranges of values are disclosed, and the notation "from n₁ ... to n₂" or "n₁-n₂" is used, where n₁ and n₂ are the numbers, then unless otherwise specified, this notation is intended to include the numbers themselves and the range between them. This range may be integral or continuous between and including the end values.

The term "deuterium enrichment" refers to the percentage of incorporation of deuterium at a given position in a molecule in the place of hydrogen. For example, deuterium enrichment of 1% at a given position means that 1% of molecules in a given sample contain deuterium at the specified position. Because the naturally occurring distribution of deuterium is about 0.0156%, deuterium enrichment at any position in a compound synthesized using non-enriched starting materials is about 0.0156%. The deuterium enrichment can be determined using conventional analytical methods known to one of ordinary skill in the art, including mass spectrometry and nuclear magnetic resonance spectroscopy.

The term "is/are deuterium," when used to describe a given position in a molecule, the symbol "D", when used to represent a given position in a drawing of a molecular structure, or the term "deutero", when used as part of a chemical name, means that the specified position is enriched with deuterium above the naturally occurring distribution of deuterium. In one embodiment deuterium enrichment is no less than about 1%, in another no less than about 5%, in another no less than about 10%, in another no less than about 20%, in another no less than about 50%, in another no less than about 70%, in another no less than about 80%, in another no less than about 90%, in another no less than about 95%, in another no less than about 96%, or in another no less than about 98% of deuterium at the specified position.

The term "isotopic enrichment" refers to the percentage of incorporation of a less prevalent isotope of an element at a given position in a molecule in the place of the more prevalent isotope of the element.

The term "non-isotopically enriched" refers to a molecule in which the percentages of the various isotopes are substantially the same as the naturally occurring percentages.

Asymmetric centers exist in the compounds disclosed herein. These centers are designated by the symbols "R" or "S," depending on the configuration of substituents around the chiral carbon atom. It should be understood that the invention encompasses all stereochemical isomeric forms, including diastereomeric, enantiomeric, and epimeric forms, as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention. Additionally, the compounds disclosed herein can exist in unsolvated as well as solvated forms with pharmaceutically acceptable solvents such as water, ethanol, and the like. In general, the solvated forms are considered equivalent to the unsolvated forms.

The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disorder" as used herein is intended to be generally synonymous, and is used interchangeably with, the terms "disease" and "condition" (as in medical condition), in that all reflect an abnormal condition of the human or animal body or of one of its parts that impairs normal functioning, is typically manifested by distinguishing signs and symptoms.

The terms "treat," "treating," and "treatment" are meant to include alleviating or abrogating a disorder or one or more of the symptoms associated with a disorder; or alleviating or eradicating the cause(s) of the disorder itself. As used herein, reference to "treatment"of a disorder is intended to include prevention. The terms "prevent," "preventing," and "prevention" refer to a method of delaying or precluding the onset of a disorder; and/or its attendant symptoms, barring a subject from acquiring a disorder or reducing a subject's risk of acquiring a disorder.

The term "therapeutically effective amount" refers to the amount of a compound that, when administered, is sufficient to prevent development of, or alleviate to some extent, one or more of the symptoms of the disorder being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

The term "subject" refers to an animal, including, but not limited to, a primate (e.g., human, monkey, chimpanzee, gorilla, and the like), rodents (e.g., rats, mice, gerbils, hamsters, ferrets, and the like), lagomorphs, swine (e.g., pig, miniature pig), equine, canine, feline, and the like. The terms "subject" and "patient" are used interchangeably herein in reference, for example, to a mammalian subject, such as a human patient.

The term "combination therapy" means the administration of two or more therapeutic agents to treat a therapeutic disorder described in the present disclosure. Such administration encompasses co-administration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the disorders described herein.

The terms "Non-oral medication" or "non-oral application" refers to any medication or application pathway that is not applied perorally through the mouth (*per os*). Non-oral medication or non-oral application comprises injections, which may be intravenous, intra-arterial, percutaneous, subcutaneous, intramuscular, intraperitoneal, or infusions, which may be intravenous, intra-arterial, percutaneous, subcutaneous, intramuscular, intraperitoneal, or application via gastric tubes, PEG (Percutaneous Endoscopic Gastrostomy) tubes, jejunal tubes, PEJ (Percutaneous Endoscopic Jejunostomy) tubes, small intestine tubes, or duodenal tubes and the like.

The term "neurotransmitter" refers to endogenous chemicals that transmit signals across a synapse from one neuron (brain cell) to another 'target' neuron. Neurotransmitters are packaged into synaptic vesicles clustered beneath the membrane in the axon terminal, on the presynaptic side of a synapse. Neurotransmitters are released into and diffuse across the synaptic cleft, where they bind to specific receptors in the membrane on the postsynaptic side of the synapse. Many neurotransmitters are synthesized from plentiful and simple precursors, such as amino acids, which are readily available from the diet and which require only a small number of biosynthetic steps to convert. Specific neurotransmitters whose levels are modulated by the compounds disclosed herein include norepinephrine and epinephrine.

Dopamine is a catecholamine with multiple roles including those as a hormone and a neurotransmitter. The brain includes several distinct dopamine systems, one of which plays a major role in reward-motivated behavior. Most types of reward increase the level of dopamine in the brain, and a variety of addictive drugs increase dopamine neuronal activity. Other brain dopamine systems are involved in motor control and in controlling the release of several other important hormones. Norepinephrine is synthesized from L-DOPA which is converted into dopamine by the enzyme aromatic L-amino acid decarboxylase (AADC; also known as DOPA decarboxylase (DDC)). The actions of dopamien are carried out via the binding to dopamine receptors.

Norepinephrine is a catecholamine with multiple roles including those as a hormone and a neurotransmitter. Medically it is used in those with severe hypotension. It does this by increasing vascular tone (tension of vascular smooth muscle) through α-adrenergic receptor activation. One of the most important functions of norepinephrine is its role as the neurotransmitter released from the sympathetic neurons to affect the heart. An increase in norepinephrine from the sympathetic nervous system increases the rate of contractions in the heart. As a stress hormone, norepinephrine affects parts of the brain, such as the amygdala, where attention and responses are controlled. Norepinephrine also underlies the fight-or-flight response, along with epinephrine, directly increasing heart rate, triggering the release of glucose from energy stores, and increasing blood flow to skeletal muscle. It increases the brain's oxygen supply. Norepinephrine is synthesized from dopamine by dopamine β-hydroxylase in the secretory granules of the medullary chromaffin cells. It is released from the adrenal medulla into the blood as a hormone, and is also a neurotransmitter in the central nervous system and sympathetic nervous system, where it is released from noradrenergic neurons in the locus coeruleus. The actions of norepinephrine are carried out via the binding to adrenergic receptors.

Epinephrine is a hormone and a neurotransmitter which acts on nearly all body tissues. Its actions vary by tissue type and tissue expression of adrenergic receptors. For example, high levels of epinephrine cause smooth muscle relaxation in the airways but causes contraction of the smooth muscle that lines most arterioles. Epinephrine acts by binding to a variety of adrenergic receptors. Epinephrine is a nonselective agonist of all adrenergic receptors, including the major subtypes α1, α2, β1, β2, and β3. Epinephrine's binding to these receptors triggers a number of metabolic changes. Binding to α-adrenergic receptors inhibits insulin secretion by the pancreas, stimulates glycogenolysis in the liver and muscle, and stimulates glycolysis in muscle. β-Adrenergic receptor binding triggers glucagon secretion in the pancreas, increased adrenocorticotropic hormone (ACTH) secretion by the pituitary gland, and increased lipolysis by adipose tissue. Together, these effects lead to increased blood glucose and fatty acids, providing substrates for energy production within cells throughout the body. Adrenaline is used to treat a number of conditions including: cardiac arrest, anaphylaxis, and superficial bleeding.

The term "neurotransmitter-mediated disorder," refers to a disorder that is characterized by abnormal or suboptimal levels of dopamine, norepinephrine, and/or epinephrine. A neurotransmitter-mediated disorder may be completely or partially mediated by modulating neurotransmitter levels. In particular, a neurotransmitter-mediated disorder is one in which modulation of neurotransmitter levels results in some effect on the underlying disorder e.g., administration of a neurotransmitter modulator results in some improvement in at least some of the patients being treated. In some embodiments the term "neurotransmitter-mediated disorder" refers to a disorder in which there is decreased synthesis, storage, release, reuptake, metabolism, or effect of dopamine, epinephrine, and/or norepinephrine, such as Parkinson's disease.

The term "neurotransmitter level modulator," refers to the ability of a compound disclosed herein to alter levels of dopamine, norepinephrine, and/or epinephrine. A modulator may increase neurotransmitter levels by acting as a biosynthetic precursor to dopamine, norepinephrine, and/or epinephrine. Such modulation may be manifest only in particular cell types or may be contingent on a particular biological event.

The term "therapeutically acceptable" refers to those compounds (or salts, prodrugs, tautomers, zwitterionic forms, etc.) which are suitable for use in contact with the tissues of patients without excessive toxicity, irritation, allergic response, immunogenecity, are commensurate with a reasonable benefit/risk ratio, and are effective for their intended use.

The term "pharmaceutically acceptable carrier," "pharmaceutically acceptable excipient," "physiologically acceptable carrier," or "physiologically acceptable excipient" refers to a pharmaceutically-acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenecity, or other problems or complications, commensurate with a reasonable benefit/risk ratio. *See,* Remington: The Science and Practice of Pharmacy, 21st Edition; Lippincott Williams & Wilkins: Philadelphia, PA, 2005; Handbook of Pharmaceutical Excipients, 5th Edition; Rowe et al., Eds., The Pharmaceutical Press and the American Pharmaceutical Association: 2005; and Handbook of Pharmaceutical Additives, 3rd Edition; Ash and Ash Eds., Gower Publishing Company: 2007; Pharmaceutical Preformulation and Formulation, Gibson Ed., CRC Press LLC: Boca Raton, FL, 2004).

The terms "active ingredient," "active compound," and "active substance" refer to a compound, which is administered, alone or in combination with one or more pharmaceutically acceptable excipients or carriers, to a subject for treating, preventing, or ameliorating one or more symptoms of a disorder.

The terms "drug," "therapeutic agent," and "chemotherapeutic agent" refer to a compound, or a pharmaceutical composition thereof, which is administered to a subject for treating, preventing, or ameliorating one or more symptoms of a disorder.

The term "release controlling excipient" refers to an excipient whose primary function is to modify the duration or place of release of the active substance from a dosage form as compared with a conventional immediate release dosage form.

The term "nonrelease controlling excipient" refers to an excipient whose primary function do not include modifying the duration or place of release of the active substance from a dosage form as compared with a conventional immediate release dosage form.

The term "groups that are easily hydrolytically or enzymatically cleavable under physiological conditions" refers to common protective groups which are used in synthesis or that are such protective groups which lead to so-called prodrugs and are known to those skilled in the art. These groups may be selected from the group comprising methyl, perdeuteromethyl, ethyl, perdeuteroethyl, propyl, perdeuteropropyl, butyl, perdeuterobutyl, C₁ to C₆-alkyl, that may be branched or unbranched, or C₅ to C₆-cycloalkyl, deuterated or partly deuterated C₁ to C₆-alkyl, that may be branched or unbranched, or deuterated or partly deuterated C₅ to C₆-cycloalkyl.

The term "prodrug" refers to a compound functional derivative of the compound as disclosed herein and is readily convertible into the parent compound in vivo. Prodrugs are often useful because, in some situations, they may be easier to administer than the parent compound. They may, for instance, be bioavailable by oral administration whereas the parent compound is not. The prodrug may also have enhanced solubility in pharmaceutical compositions over the parent compound. A prodrug may be converted into the parent drug by various mechanisms, including enzymatic processes and metabolic hydrolysis. See Harper, Progress in Drug Research 1962, 4, 221-294; Morozowich et al. in "Design of Biopharmaceutical Properties through Prodrugs and Analogs," Roche Ed., APHA Acad. Pharm. Sci. 1977; "Bioreversible Carriers in Drug in Drug Design, Theory and Application," Roche Ed., APHA Acad. Pharm. Sci. 1987; "Design of Prodrugs," Bundgaard, Elsevier, 1985; Wang et al., Curr. Pharm. Design 1999, 5, 265-287; Pauletti et al., Adv. Drug. Delivery Rev. 1997, 27, 235-256; Mizen et al., Pharm. Biotech. 1998, 11, 345-365; Gaignault et al., Pract. Med. Chem. 1996, 671-696; Asgharnejad in "Transport Processes in Pharmaceutical Systems," Amidon et al., Ed., Marcell Dekker, 185-218, 2000; Balant et al., Eur. J. Drug Metab. Pharmacokinet. 1990, 15, 143-53; Balimane and Sinko, Adv. Drug Delivery Rev. 1999, 39, 183-209; Browne, Clin. Neuropharmacol. 1997, 20, 1-12; Bundgaard, Arch. Pharm. Chem. 1979, 86, 1-39; Bundgaard, Controlled Drug Delivery 1987, 17, 179-96; Bundgaard, Adv. Drug Delivery Rev.1992, 8, 1-38; Fleisher et al., Adv. Drug Delivery Rev. 1996, 19, 115-130; Fleisher et al., Methods Enzymol. 1985, 112, 360-381; Farquhar et al., J. Pharm. Sci. 1983, 72, 324-325; Freeman et al., J. Chem. Soc., Chem. Commun. 1991, 875-877; Friis and Bundgaard, Eur. J. Pharm. Sci. 1996, 4, 49-59; Gangwar et al., Des. Biopharm. Prop. Prodrugs Analogs, 1977, 409-421; Nathwani and Wood, Drugs 1993, 45, 866-94; Sinhababu and Thakker, Adv. Drug Delivery Rev. 1996, 19, 241-273; Stella et al., Drugs 1985, 29, 455-73; Tan et al., Adv. Drug Delivery Rev. 1999, 39, 117-151; Taylor, Adv. Drug Delivery Rev. 1996, 19, 131-148; Valentino and Borchardt, Drug Discovery Today 1997, 2, 148-155; Wiebe and Knaus, Adv. Drug Delivery Rev. 1999, 39, 63-80; Waller et al., Br. J. Clin. Pharmac. 1989, 28, 497-507.

The compounds disclosed herein can exist as therapeutically acceptable salts. The term "therapeutically acceptable salt," as used herein, represents salts or zwitterionic forms of the compounds disclosed herein which are therapeutically acceptable as defined herein. The salts can be prepared during the final isolation and purification of the compounds or separately by reacting the appropriate compound with a suitable acid or base.Therapeutically acceptable salts include acid and basic addition salts. For a more complete discussion of the preparation and selection of salts, refer to "Handbook of Pharmaceutical Salts, Properties, and Use," Stah and Wermuth, Ed.;(Wiley-VCH and VHCA, Zurich, 2002) and Berge et al., J. Pharm. Sci. 1977, 66, 1-19.

Suitable acids for use in the preparation of pharmaceutically acceptable salts include, but are not limited to, acetic acid, 2,2-dichloroacetic acid, acylated amino acids, adipic acid, alginic acid, ascorbic acid, L-aspartic acid, benzenesulfonic acid, benzoic acid, 4-acetamidobenzoic acid, boric acid, (+)-camphoric acid, camphorsulfonic acid, (+)-(1S)-camphor-10-sulfonic acid, capric acid, caproic acid, caprylic acid, cinnamic acid, citric acid, cyclamic acid, cyclohexanesulfamic acid, dodecylsulfuric acid, ethane-1,2-disulfonic acid, ethanesulfonic acid, 2-hydroxy-ethanesulfonic acid, formic acid, fumaric acid, galactaric acid, gentisic acid, glucoheptonic acid, D-gluconic acid, D-glucuronic acid, L-glutamic acid, α-oxo-glutaric acid, glycolic acid, hippuric acid, hydrobromic acid, hydrochloric acid, hydroiodic acid, (+)-L-lactic acid, (±)-DL-lactic acid, lactobionic acid, lauric acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotinic acid, nitric acid, oleic acid, orotic acid, oxalic acid, palmitic acid, pamoic acid, perchloric acid, phosphoric acid, L-pyroglutamic acid, saccharic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, undecylenic acid, and valeric acid.

For the production of the physiologically acceptable salts of the compounds disclosed herein, the usual physiologically acceptable inorganic and organic acids such as hydrochloric acid, hydrobromic acid, phosphoric acid, sulfuric acid, oxalic acid, maleic acid, fumaric acid, lactic acid, tartaric acid, malic acid, citric acid, salicylic acid, adipic acid and benzoic acid can be used, as well as salts with suitable zwitterions (like lysinate and aspartate). Additional acids that can be used are described, for example, in Fortschritte der Arzneimittelforschung, Vol. 10, pp. 224-225, Birkhäuser Publishers, Basel and Stuttgart, 1966, and Journal of Pharmaceutical Sciences, Vol. 66, pp. 1-5 (1977).

The acid addition salts are usually obtained in a way known in and of itself by mixing the free base or solutions thereof with the corresponding acid or solutions thereof in an organic solvent, for example, a lower alcohol, such as methanol, ethanol, n-propanol or isopropanol or a lower ketone such as acetone, methyl ethyl ketone or methyl isobutyl ketone or an ether such as diethyl ether, tetrahydrofuran or dioxane. For better crystal precipitation, mixtures of the named solvents can also be used. In addition, physiologically acceptable aqueous solutions of acid addition salts of the compounds used according to the invention can be produced there from in an aqueous acid solution.

The acid addition salts of the compounds disclosed herein can be converted to the free base in a way known in and of itself, e.g., with alkalis or ion exchangers. Additional salts can be obtained from the free base by reaction with inorganic or organic acids, particularly those which are suitable for the formation of salts that can be employed therapeutically. These or also other salts of the new compound, such as, e.g., the picrate, may also serve for purification of the free base by converting the free base into a salt, separating this salt, and again releasing the base from the salt.

Suitable bases for use in the preparation of pharmaceutically acceptable salts, including, but not limited to, inorganic bases, such as magnesium hydroxide, calcium hydroxide, potassium hydroxide, zinc hydroxide, or sodium hydroxide; and organic bases, such as primary, secondary, tertiary, and quaternary, aliphatic and aromatic amines, including L-arginine, benethamine, benzathine, choline, deanol, diethanolamine, diethylamine, dimethylamine, dipropylamine, diisopropylamine, 2-(diethylamino)-ethanol, ethanolamine, ethylamine, ethylenediamine, isopropylamine, N-methyl-glucamine, hydrabamine, 1H-imidazole, L-lysine, morpholine, 4-(2-hydroxyethyl)-morpholine, methylamine, piperidine, piperazine, propylamine, pyrrolidine, 1-(2-hydroxyethyl)-pyrrolidine, pyridine, quinuclidine, quinoline, isoquinoline, secondary amines, triethanolamine, trimethylamine, triethylamine, N-methyl-D-glucamine, 2-amino-2-(hydroxymethyl)-1,3-propanediol, and tromethamine.

While it may be possible for the compounds of the subject invention to be administered as the raw chemical, it is also possible to present them as a pharmaceutical composition. Accordingly, provided herein are pharmaceutical compositions which comprise one or more of certain compounds disclosed herein, or one or more pharmaceutically acceptable salts, prodrugs, or solvates thereof, together with one or more pharmaceutically acceptable carriers thereof and optionally one or more other therapeutic ingredients. Proper formulation is dependent upon the route of administration chosen. Any of the well-known techniques, carriers, and excipients may be used as suitable and as understood in the art; *e.g.,* in Remington's Pharmaceutical Sciences. The pharmaceutical compositions disclosed herein may be manufactured in any manner known in the art, *e.g.,* by means of conventional mixing, dissolving, granulating, dragee-making, levigating, emulsifying, encapsulating, entrapping or compression processes. The pharmaceutical compositions may also be formulated as a modified release dosage form, including delayed-, extended-, prolonged-, sustained-, pulsatile-, controlled-, accelerated- and fast-, targeted-, programmed-release, and gastric retention dosage forms. These dosage forms can be prepared according to conventional methods and techniques known to those skilled in the art (*see,* Remington: The Science and Practice of Pharmacy, supra; Modified-Release Drug Deliver Technology, Rathbone et al., Eds., Drugs and the Pharmaceutical Science, Marcel Dekker, Inc.: New York, NY, 2002; Vol. 126; Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 1991; Ullmann's Enzyklopädie [Encyclopedia] (5th ed.) A 19, 241-271; Voigt, Pharmazeutische Technologie [Pharmaceutical Technology], Berlin: Ullstein Mosby 1995).

The compositions include those suitable for oral, non-oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The compositions may conveniently be presented in unit dosage form and may be prepared by any of the methods well known in the art of pharmacy. Typically, these methods include the step of bringing into association a compound of the subject invention or a pharmaceutically salt, prodrug, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

The compositions include those suitable for oral or non-oral administration. The compositions may conveniently be presented in unit dosage form and may be prepared by any of the methods well known in the art of pharmacy. Typically, these methods include the step of bringing into association a compound of the subject invention or a pharmaceutically salt, prodrug, or solvate thereof ("active ingredient") with the carrier which constitutes one or more accessory ingredients. In general, the compositions are prepared by uniformly and intimately bringing into association the active ingredient with liquid carriers or finely divided solid carriers or both and then, if necessary, shaping the product into the desired formulation.

Formulations of the compounds disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Solutions or suspensions containing the active substance used according to the invention may additionally contain agents that improve taste, such as saccharin, cyclamate or sugar, as well as, e.g., taste enhancers such as vanilla or orange extract. They may also contain suspension adjuvants such as sodium carboxymethylcellulose or preservatives such as p-hydroxybenzoate. Capsules containing active substances can be produced, for example, by mixing the active substance with an inert vehicle such as lactose or sorbitol and encapsulating this mixture in gelatin capsules. Suitable suppositories can be produced, for example, by mixing with vehicle agents provided therefore, such as neutral fats or polyethylene glycol or derivatives thereof.

In certain embodiments, diluents are selected from the group consisting of mannitol powder, spray dried mannitol, microcrystalline cellulose, lactose, dicalcium phosphate, tricalcium phosphate, starch, pregelatinized starch, compressible sugars, silicified microcrystalline cellulose, and calcium carbonate.

In certain embodiments, surfactants are selected from the group consisting of Tween 80, sodium lauryl sulfate, and docusate sodium.

In certain embodiments, binders are selected from the group consisting of povidone (PVP) K29/32, hydroxypropylcellulose (HPC), hydroxypropylmethylcellulose (HPMC), ethylcellulose (EC), corn starch, pregelatinized starch, gelatin, and sugar.

In certain embodiments, lubricants are selected from the group consisting of magnesium stearate, stearic acid, sodium stearyl fumarate, calcium stearate, hydrogenated vegetable oil, mineral oil, polyethylene glycol, polyethylene glycol 4000-6000, talc, and glyceryl behenate.

In certain embodiments, sustained release polymers are selected from the group consisting of POLYOX® (poly (ethylene oxide), POLYOX® N60K grade, Kollidon® SR, HPMC, HPMC (high viscosity), HPC, HPC (high viscosity), and Carbopol®.

In certain embodiments, extended/controlled release coating are selected from a group of ethylcellulose polymers, such as ETHOCEL™ and Surelease® Aqueous Ethylcellulose Dispersions.

In certain embodiments, antioxidants are selected from a group consisting of butylated hydroxyanisole (BHA), butylated hydroxytoluene (BHT), sodium ascorbate, and α-tocopherol.

In certain embodiments, tablet coatings are selected from the group of Opadry® 200, Opadry® II, Opadry® fx, Opadry® amb, Opaglos® 2, Opadry® tm, Opadry®, Opadry® NS, Opalux®, Opatint®, Opaspray®, Nutraficient®.

Preferred unit dosage formulations are those containing an effective dose, as herein below recited, or an appropriate fraction thereof, of the active ingredient.

Compounds may be administered orally at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

The compounds may be formulated for non-oral and/or parenteral administration by injection, e.g., by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for non-oral and/or parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Solvents can be used for the preparation of the pharmaceutical composition in order to obtain the required injectability criteria. Solvents can be selected from alcohols such as ethanol, isopropanol, methanol or n-propanol. Solvents can also be selected from polyols such as propyleneglycol, glycerol, mannitol, maltitol or from cyclodextrin derivatives such as sulfobutylether β cyclodextrin or hydroxypropyl β cyclodextrin. Further solvents can be selected from the group of polyethers.

Surfactants can also be used for the preparation of the pharmaceutical composition according to the present invention. Surfactants can be used from the group of polyoxyethylene sorbitan fatty acid esters such as polysorbate 80 or polysorbate 20.

Physiologically acceptable sugars such as dextran, mannitol or glucose can be used for the preparation of the pharmaceutical composition according to the invention.

The pH of the pharmaceutical composition plays a crucial role for the preparation. Levodopa is poorly dissolved at neutral pH. Best solubility of levodopa is achieved at a lower pH between 3 and 6. Whereas a pH below 4 could cause blood vessel irritations and thrombophlebitis. Therefore a pH in the range of 4 to 6 is preferred for the pharmaceutical composition according to the present invention whereas a range of 4.5 to 5.5 is even more preferred. In order to achieve the required pH values, pH regulators are used during the preparation of the pharmaceutical composition. pH regulators can be selected from the usual physiologically acceptable inorganic and organic acids such as hydrochloric acid, hydrobromic acid, phosphoric acid, sulfuric acid, oxalic acid, maleic acid, fumaric acid, lactic acid, tartaric acid, malic acid, citric acid, salicylic acid, adipic acid and benzoic acid. In certain embodiments pH regulators are selected from weak acids such as hydrochloric acid and acetic acid.

The pharmaceutical compositions may include antioxidants. Antioxidants can be selected from the group of acids and their salts, vitamins and vitamin derivatives, amino acids, sulfites, free phenolic radical scavengers, as well as thioctic acid.

Solutions or suspensions containing the active substances used according to the invention may additionally contain suspension adjuvants such as sodium carboxymethylcellulose or preservatives such as p-hydroxybenzoate.

The pharmaceutical composition according to the present invention can also be used in infusion pumps for subcutaneous or intrathekal application. Different infusion systems have been described in the literature relaying on two different ways of application. Either the pharmaceutical composition can be used as infusion solution in a cassette or in a bottle connected with an adapter to the pump system.

The pharmaceutical composition according to the invention can also be used in the gastric pumps known in the art.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds may also be formulated in rectal compositions such as suppositories or retention enemas, *e.g.,* containing conventional suppository bases such as cocoa butter, polyethylene glycol, or other glycerides.

Certain compounds disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semiliquid preparations suitable for penetration through the skin to the site of inflammation such as gels, liniments, lotions, creams, ointments or pastes, and drops suitable for administration to the eye, ear or nose.

For administration by inhalation, compounds may be delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

Preferred unit dosage formulations are those containing an effective dose, as herein below recited, or an appropriate fraction thereof, of the active ingredient.

Compounds may be administered orally non-oral and/or via injection at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

In order to obtain the desired effect, the dose of active principle can vary between 100 and 1500 mg per day in divided doses.

Each single dose can contain from 50 to 1000 mg of active principle, in combination with a pharmaceutical vehicle. This single dose can be administered 1 to 4 times daily.

The amount of active ingredient that may be combined with the carrier materials to produce a single dosage form will vary depending upon the host treated and the particular mode of administration.

The compounds can be administered in various modes, *e.g.* non-orally, orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the disorder being treated. Also, the route of administration may vary depending on the disorder and its severity.

In the case wherein the patient's condition does not improve, upon the doctor's discretion the administration of the compounds may be administered chronically, that is, for an extended period of time, including throughout the duration of the patient's life in order to ameliorate or otherwise control or limit the symptoms of the patient's disorder.

In the case wherein the patient's status does improve, upon the doctor's discretion the administration of the compounds may be given continuously or temporarily suspended for a certain length of time (i.e., a "drug holiday").

Once improvement of the patient's conditions has occurred, a maintenance dose is administered if necessary. Subsequently, the dosage or the frequency of administration, or both, can be reduced, as a function of the symptoms, to a level at which the improved disorder is retained. Patients can, however, require intermittent treatment on a long-term basis upon any recurrence of symptoms.

Disclosed herein are methods of treating a neurotransmitter kinase-mediated disorder comprising administering to a subject having or suspected to have such a disorder, a therapeutically effective amount of a compound as disclosed herein or a pharmaceutically acceptable salt, solvate, or prodrug thereof.

Neurotransmitter-mediated disorders, include, but are not limited to, Parkinson's disease, restless leg syndrome, dystonia, for inhibiting prolactin secretion, for stimulating the release of growth hormones, for the treatment of neurological symptoms of chronic manganese intoxication, amyotrophic lateral scleroses, and multiple system atrophy.

In certain embodiments, a method of treating a neurotransmitter-mediated disorder comprises administering to the subject a therapeutically effective amount of a compound of as disclosed herein, or a pharmaceutically acceptable salt, solvate, or prodrug thereof, so as to affect: (1) decreased inter-individual variation in plasma levels of the compound or a metabolite thereof; (2) increased average plasma levels of the compound or decreased average plasma levels of at least one metabolite of the compound per dosage unit; (3) decreased inhibition of, and/or metabolism by at least one cytochrome P₄₅₀ or monoamine oxidase isoform in the subject; (4) decreased metabolism via at least one polymorphically-expressed cytochrome P₄₅₀ isoform in the subject; (5) at least one statistically-significantly improved disorder-control and/or disorder-eradication endpoint; (6) an improved clinical effect during the treatment of the disorder, (7) prevention of recurrence, or delay of decline or appearance, of abnormal alimentary or hepatic parameters as the primary clinical benefit, or (8) reduction or elimination of deleterious changes in any diagnostic hepatobiliary function endpoints, as compared to the corresponding non-isotopically enriched compound.

In certain embodiments, inter-individual variation in plasma levels of the compounds as disclosed herein, or metabolites thereof, is decreased; average plasma levels of the compound as disclosed herein are increased; average plasma levels of a metabolite of the compound as disclosed herein are decreased; inhibition of a cytochrome P₄₅₀ or monoamine oxidase isoform by a compound as disclosed herein is decreased; or metabolism of the compound as disclosed herein by at least one polymorphically-expressed cytochrome P₄₅₀ isoform is decreased; by greater than about 5%, greater than about 10%, greater than about 20%, greater than about 30%, greater than about 40%, or by greater than about 50% as compared to the corresponding non-isotopically enriched compound.

Plasma levels of the compound as disclosed herein, or metabolites thereof, may be measured using the methods known in the art.

Examples of cytochrome P₄₅₀ isoforms in a mammalian subject include, but are not limited to, CYP1A1, CYP1A2, CYP1B1, CYP2A6, CYP2A13, CYP2B6, CYP2C8, CYP2C9, CYP2C18, CYP2C19, CYP2D6, CYP2E1, CYP2G1, CYP2J2, CYP2R1, CYP2S1, CYP3A4, CYP3A5, CYP3A5P1, CYP3A5P2, CYP3A7, CYP4A11, CYP4B1, CYP4F2, CYP4F3, CYP4F8, CYP4F11, CYP4F12, CYP4X1, CYP4Z1, CYP5A1, CYP7A1, CYP7B1, CYP8A1, CYP8B1, CYP11A1, CYP11B1, CYP11B2, CYP17, CYP19, CYP21, CYP24, CYP26A1, CYP26B1, CYP27A1, CYP27B1, CYP39, CYP46, and CYP51.

Examples of monoamine oxidase isoforms in a mammalian subject include, but are not limited to, MAO_{A}, and MAO_{B}.

The inhibition of the cytochrome P₄₅₀ isoform is measured by the method of Ko et al. (British Journal of Clinical Pharmacology, 2000, 49, 343-351). The inhibition of the MAO_{A} isoform is measured by the method of Weyler et al. (J. Biol Chem. 1985, 260, 13199-13207). The inhibition of the MAO_{B} isoform is measured by the method of Uebelhack et al. (Pharmacopsychiatry, 1998, 31, 187-192).

Examples of polymorphically-expressed cytochrome P₄₅₀ isoforms in a mammalian subject include, but are not limited to, CYP2C8, CYP2C9, CYP2C19, and CYP2D6.

The metabolic activities of liver microsomes, cytochrome P₄₅₀ isoforms, and monoamine oxidase isoforms are measured by the methods described herein.

Examples of improved disorder-control and/or disorder-eradication endpoints, or improved clinical effects include, but are not limited to:
a. improved Unified Parkinson's Disease Rating Scale scores;
b. improved Abnormal Involuntary Movement Scale scores;
c. improved Goetz Dyskinesia Rating Scale scores;
d. improved Unified Dyskinesia Rating Scale scores;
e. improved PDQ-39 Parkinson's Disease Questionnaire scores; and
f. improved Global Primate Dyskinesia Rating Scale scores.

Examples of diagnostic hepatobiliary function endpoints include, but are not limited to, alanine aminotransferase ("ALT"), serum glutamic-pyruvic transaminase ("SGPT"), aspartate aminotransferase ("AST" or "SGOT"), ALT/AST ratios, serum aldolase, alkaline phosphatase ("ALP"), ammonia levels, bilirubin, gamma-glutamyl transpeptidase ("GGTP," "γ-GTP," or "GGT"), leucine aminopeptidase ("LAP"), liver biopsy, liver ultrasonography, liver nuclear scan, 5'-nucleotidase, and blood protein. Hepatobiliary endpoints are compared to the stated normal levels as given in "Diagnostic and Laboratory Test Reference", 4th edition, Mosby, 1999. These assays are run by accredited laboratories according to standard protocol.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

### Combination Therapy

The compounds disclosed herein may also be combined or used in combination with other agents useful in the treatment of neurotransmitter kinase-mediated disorders. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced).

Such other agents, adjuvants, or drugs, may be administered, by a route and in an amount commonly used therefor, simultaneously or sequentially with a compound as disclosed herein. When a compound as disclosed herein is used contemporaneously with one or more other drugs, a pharmaceutical composition containing such other drugs in addition to the compound disclosed herein may be utilized, but is not required.

In certain embodiments, the compounds disclosed herein can be combined with one or more sympathomimetic agents selected from the group consisting of epinephrine, norepinephrine, phenylephrine, dobutamine, dopamine, ephedrine, midodrine, and amezinium.

In certain embodiments, the compounds disclosed herein can be combined with one or more S-alkylisothiouronium derivatives selected from the group consisting of difetur and izoturon.

In certain embodiments, the compounds disclosed herein can be combined with one or more glucocorticoids selected from the group consisting of hydrocortisone, prednisone, prednisolone, dexamethasone, and betamethasone.

In certain embodiments, the compounds disclosed herein can be combined with one or more analeptics selected from the group consisting of bemegride, caffeine, camphora, and cordiamine.

In certain embodiments, the compounds disclosed herein can be combined with one or more psychotropics selected from the group consisting of amphetamine, atomoxetine, bupropion, duloxetine, methamphetamine, methylphenidate, reboxetine, and venlafaxine.

In certain embodiments, the compounds disclosed herein can be combined with one or more positive inotropic agents selected from the group consisting of cardiac glycosides, strophantin K, corglycon, digoxin, amrinone, and milrinone.

In certain embodiments, the compounds disclosed herein can be combined with one or more antihypotensive agents selected from the group consisting of angiotensinamide, indomethacin, oxilofrine, potassium chloride, and yohimbine.

In certain embodiments, the compounds disclosed herein can be combined with one or more L-aromatic-amino acid decarboxylase inhibitor selected from the group consisting of benserazide, carbidopa, methyldopa, and α-difluoromethyl-DOPA.

In certain embodiments, the compounds disclosed herein can be combined with one or more catechol-O-methyltransferase inhibitors selected from the group consisting of entacapone, tolcapone, and nitecapone.

In certain embodiments, the compounds disclosed herein can be combined with one or more monoamine oxidase inhibitors selected from the group consisting of isocarboxazid, isoniazid, nialamide, phenelzine, tranylcypromine, moclobemide, pirlindole, toloxatone, rasagiline, and selegiline.

In certain embodiments, the compounds disclosed herein can be combined with one or more 5-HT_{2A} inverse agonist selected from the group consisting of pimvaserin.

Thus, in another aspect, certain embodiments provide methods for treating neurotransmitter kinase-mediated disorders in a human or animal subject in need of such treatment comprising administering to said subject an amount of a compound disclosed herein effective to reduce or prevent said disorder in the subject, in combination with at least one additional agent for the treatment of said disorder that is known in the art. In a related aspect, certain embodiments provide therapeutic compositions comprising at least one compound disclosed herein in combination with one or more additional agents for the treatment of neurotransmitter kinase-mediated disorders.

### General Synthetic Methods for Preparing Compounds

Isotopic hydrogen can be introduced into a compound as disclosed herein by synthetic techniques that employ deuterated reagents, whereby incorporation rates are pre-determined; and/or by exchange techniques, wherein incorporation rates are determined by equilibrium conditions, and may be highly variable depending on the reaction conditions. Synthetic techniques, where deuterium is directly and specifically inserted by deuterated reagents of known isotopic content, may yield high deuterium abundance, but can be limited by the chemistry required. Exchange techniques, on the other hand, may yield lower deuterium incorporation, often with the isotope being distributed over many sites on the molecule.

The compounds as disclosed herein can be prepared by methods known to one of skill in the art and routine modifications thereof, and/or following procedures similar to those described in the Example section herein and routine modifications thereof, and/or procedures found in US Patent No. 8,168,820, US Patent No. 8,247,603, WO 2004056724, WO 2007093450, and WO 2014122184, which are hereby incorporated in their entirety, and references cited therein and routine modifications thereof.

### Examples

The following examples shall be understood only as one preferred embodiment of the invention. It is not intended to limit the present invention to the scope of the given examples.

The following examples provide possible recipes for an injectable solution of the pharmaceutical composition according to the present invention with and without the additional use of carbidopa and cytoprotective compounds.

### Example 1

### Preparation of a pharmaceutical composition containing levodopa (L-DOPA) and apomorphine.

### Stock solution 1:

Levodopa 50 mg/ml and Apomorphine 25 mg/ml

| **Substance** | **Weight** |
|---|---|
| Levodopa | 5 g |
| Hydrochloric acid | 15.5 g |
| Sodium pyrosulfite | 0.5 g |
| Apomorphine | 2.5 g |
| Propylene glycol | 30 g |
| Polysorbate 80 | 0.3 g |
| Water | Filled to 100 ml |

Levodopa is weighed and dissolved in a beaker containing a HCl solution while stirring. The sodium pyrosulfite is weighed in a small beaker and dissolved by adding 2 ml of sterile water. The dissolved pyrosulfite solution is taken up by a pipette and added to the solution containing levodopa. In the meanwhile propylene glycol and polysorbate 80 are weighed and dis-solved with water in a medium-sized beaker. Apomorphine is added to the propylene glycol / polysorbate 80 solution after adjusting the pH. The solution containing apomorphine is stirred on a magnetic stirrer until all substances are completely dissolved. After apomorphine is dissolved the solution is added to the beaker containing the levodopa solution.

The solution is filled up to 100 ml with sterile water and stirred on a magnetic stirrer. The combined solutions are further stirred until a completely homogenized solution is prepared.

### Stock solution 1:

Levodopa 10 mg/ml and Apomorphine 5 mg/ml

| **Substance** | **Weight** |
|---|---|
| Levodopa and apomorphine | 20 ml of the stock solution 1 |
| TRIS Buffer | 3 ml |
| Glucose | 25 mg/ml add to 100 ml |

For a ready-to-use solution in order to perform injections or use the solution in infusion pumps, TRIS buffer is added to the solution containing levodopa and apomorphine. Glucose is slowly added under stirring, up to a total volume of 100 ml. The pH is checked before the final solution is sterile filtered and used.

Instead of using the solvents propylenglycol (30%) and polysorbate (0.3%) to dissolve apomorphine, either the solvent sulfobutylether β cyclodextrin (20%) or the solvent hydroxypropyl β cyclodextrin (20%) can be used as well.

### Example 2

### Preparation of a pharmaceutical composition containing levodopa and apomorphine and carbidopa.

### Stock solution 2:

| **Substance** | **Weight** |
|---|---|
| Levodopa | 5 g |
| Hydrochloric acid | 15.5 g |
| Sodium pyrosulfite | 0.5 g |
| Apomorphine | 2.5 g |
| Propylene glycol | 30 g |
| Polysorbate 80 | 0.3 g |
| Carbidopa | 1 g |
| Water | Filled to 100 ml |

Levodopa is weighed and dissolved in a beaker containing a HCl solution while stirring. The sodium pyrosulfite is weighed in a small beaker and dissolved by adding 2 ml of sterile water. The dissolved pyrosulfite solution is taken up by a pipette and added to the solution containing levodopa. In the meanwhile propylene glycol and polysorbate 80 are weighed and dissolved with water in a medium-sized beaker. Apomorphine is added to the propylene glycol / polysorbate 80 solution after adjusting the pH. The solution containing apomorphine is stirred on a magnetic stirrer until all substances are completely dissolved. After apomorphine is dissolved the solution is added to the beaker containing the levodopa solution. The combined solutions are further stirred until a completely homogenized solution is prepared. The pH is checked.

Carbidopa is weighed in dissolved in a beaker containing sterile water and stirred until completely dissolved. Heating might be required. Afterwards the carbidopa solution is added to the solution containing levodopa and apomorphine.

The solution is filled up to 100 ml with sterile water and stirred on a magnetic stirrer. The final solution is filtered before use.

Instead of using the solvents propylenglycol (30%) and polysorbate (0.3%) to dissolve apomorphine, either the solvent sulfobutylether β cyclodextrin (20%) or the solvent hydroxypropyl β cyclodextrin (20%) can be used as well.

### Solution 2:

Levodopa 10 mg/ml, Apomorphine 5 mg/ml and Carbidopa 2 mg/ml

| **Substance** | **Weight** |
|---|---|
| Levodopa, apomorphine, and carbidopa | 20 ml of the 50 mg/ml stock solution 2 |
| TRIS Buffer | 3 ml |
| Glucose | 25 mg/ml add to 100 ml |

For a ready-to-use solution in order to perform injections or use the solution in infusion pumps, TRIS buffer is added to the solution containing levodopa, apomorphine and carbidopa. Glucose is slowly added under stirring, up to a total volume of 100 ml. The final solution is sterile filtered before use.

### Example 3

### Preparation of a pharmaceutical composition comprising a deuterated form of L-DOPA namely S/S-2-amino-2,3-dideutero-3-(3,4-dihydroxyphenyl) propionic acid (α,β-D2-L-DOPA), apomorphine and carbidopa.

The stock solution 3 is prepared as the stock solution 2 described in example 2 containing 50 mg/ml α,β-D2-L-DOPA, 25 mg/ml apomorphine and 10 mg/ml carbidopa.

Apomorphine is dissolved in the solvent hydroxypropyl β cyclodextrin (20%) instead of propylenglycol and polysorbate 80 as described in example 2 before being mixed with the solution containing the dissolved α,β-D2-L-DOPA.

Afterwards, a working solution 3 is diluted having an end concentration of 10 mg/ml α,β-D2-L-DOPA, 5 mg/ml apomorphine and 2 mg/ml carbidopa. The diluted solution is prepared as described in example 2 and also sterile filtered before use.

### Example 4

### Preparation of a pharmaceutical composition containing apomorphine and carbidopa together with a deuterated form of L-DOPA which is not completely deuterated at one position indicated by an asterix (β*).

The preparation of the stock solution 4 containing 50 mg/ml L-2-Amino-2,3,3-trideutero-3-(3,4-dihydroxyphenyl) propionic acid (α,β,β*-D3-L-DOPA), 25 mg/ml apomorphine and 10 mg/ml carbidopa is in accordance to the description found in example 2. The only difference is the solvent used. Instead of using propylenglycol and polysorbate 80 as described in example 2, sulfobutylether β cyclodextrin is used to dissolve apomorphine.

The α,β,β*-D3-L-DOPA has a deuterium enrichment of 90 % in the βR position. α,β,β*-D3-L-DOPA is obtained by mixing 10 mol% L-2-Amino-2,3(S)-dideutero-3-(3,4-dihydroxyphenyl) propionic acid with 90 mol% L-2-Amino-2,3,3-trideutero-3-(3,4-dihydroxyphenyl) propionic acid (deuterium enrich-ment >98 % in all three positions).

Before using the prepared pharmaceutical composition, a working solution 4 is diluted from stock solution 4 having an end concentration of 10 mg/ml α,β,β*-D3-L-DOPA, 5 mg/ml apomorphine and 2 mg/ml carbidopa. The diluted solution is prepared as described in example 2 and also sterile filtered before use.

### Example 5

### Preparation of a pharmaceutical composition comprising,DP-102, a deuterated form of L-DOPA, namely L-2-Amino-2,3,3-trideutero-3-(3,4- dihydroxyphenyl) propionic acid (α,β,β-D3-L-DOPA), apomorphine and carbidopa.

The stock solution 3 is prepared as the stock solution 2 described in example 2 containing 50 mg/ml DP-102, 25 mg/ml apomorphine and 10 mg/ml carbidopa.

Apomorphine is dissolved in the solvent hydroxypropyl β cyclodextrin (20%) instead of propylenglycol and polysorbate 80 as described in example 2 before being mixed with the solution containing the dissolved α,β,β-D3-L-DOPA. Afterwards a working solution 3 is diluted having an end concentration of 10 mg/ml α,β,β-D3-L-DOPA, 5 mg/ml apomorphine and 2 mg/ml carbidopa. The diluted solution is prepared as described in example 2 and also sterile filtered before use.

### Example 6

### Preparation of a pharmaceutical composition comprising,DP-102, a deuterated form of L-DOPA, namely L-2-Amino-2,3,3-trideutero-3-(3,4-dihydroxyphenyl) propionic acid (α,β,β-D3-L-DOPA), apomorphine, carbidopa, and cysteine.

The stock solution 3 is prepared as the stock solution 2 described in example 2 containing 50 mg/ml DP-102, 25 mg/ml apomorphine and 10 mg/ml carbidopa.

Apomorphine is dissolved in the solvent hydroxypropyl β cyclodextrin (20%) instead of propylene glycol and polysorbate 80 as described in example 2 before being mixed with the solution containing the dissolved α,β,β-D3-L-DOPA.

Afterwards a working solution 3 is diluted having an end concentration of 10 mg/ml α,β,β-D3-L-DOPA, 5 mg/ml apomorphine and 2 mg/ml carbidopa. Finally, cysteine is added to a final concentration of 1.3 % (w/v). The diluted solution is prepared as described in example 2 and also sterile filtered before use.

### Example 7

### MPTP monkey model of Parkinson's disease.

### Experimental details:

Macaques (Macaca fascicularis) were rendered parkinsonian by repeated daily injections of MPTP hydrochloride (0.2 mg/kg, i.v.) until parkinsonian signs appeared. The degree of parkinsonism was assessed using a validated parkinsonian macaque clinical scale which rates the following symptoms of parkinsonian disability: tremor (0-3), variations in the general level of activity (0-3), body posture (flexion of spine; 0-3), vocalization (0-2), freezing (0-2), frequency of arm movements (reaching for food, 0-3 for each upper limb), and rigidity (0-3 for each upper limb).

The maximum parkinsonian disability score was 25. MPTP administration was stopped when a score of 6 was reached on the above scale (usually day 13 - 17). The macaques were left untreated, for a minimum of 6 weeks, until their parkinsonism became stable.

This study assessed the test item in a within-subject design with an intra-individually escalating dosage regimen.

Eight animals were randomly assigned to one of two treatment sequence groups (1: AB, 2: BA). Each sequence started with a vehicle treatment, followed by a treatment period (A or B), another vehicle treatment and a 2nd treatment period (B or A).

In each treatment period (A or B) up to 9 escalating doses were administered for 5 days followed by a sixth day of administration for behavioural assessment.

Tremor was assessed for 3 hours post L-DOPA administration for 10 minutes every 30 minutes, by post hoc analysis of video recordings by a trained assessor blinded to the study treatments. (Tremor score: 0 = absent; 1 = present). The tremor scores shown in Figure 1 represent the sum of the individual scores in the assessment period.

The anti-parkinsonian action was evaluated based upon the "ON" state of the animals. The ON state was defined as being when bradykinesia was absent, i.e. score equal to zero. As the behaviour was rated during six 10 min intervals, the ON state resulted in a score ranging from 0 to 6 intervals with zero bradykinesia. ON was associated with dyskinesia of varying severity was defined as follows; "good" quality ON represented the number of scores for which bradykinesia was zero whilst dyskinesia was either absent, mild or moderate (0-2). "Bad quality" ON represented number of scores for which bradykinesia was zero whilst dyskinesia was either marked or severe (3-4).

### Result:

The mean tremor ratings for all L-DOPA doses displayed are higher or ob- served at lower Good ON levels compared to DP-102 (α,β,β-D3-L-DOPA). Further aspects and embodiments of the present invention are set out in the numbered clauses below:
1. A pharmaceutical composition comprising
   i) a dopamine agonist; and
   ii) a L-DOPA derivative in combination in form of a liquid preparation.
2. The pharmaceutical composition, according to clause 1, in form of a non-orally applicable liquid preparation.
3. The pharmaceutical composition, according to clause 1 or 2, further comprising pharmaceutically acceptable adjuvants and additives from the group of solvents, sugars or pH regulators.
4. The pharmaceutical composition, according to a least one of the preceding clauses, wherein the dopamine agonist is selected from the group comprising apomorphine, ropinirole, rotigotine, pramipexole, and piribedile.
5. The pharmaceutical composition, according to a least one of the preceding clauses, wherein the L-DOPA derivative is selected from L-DOPA, selectively and/or partially deuterated L-DOPA derivatives, as well as physiologically acceptable salts of the aforementioned L-DOPA derivatives.
6. The pharmaceutical composition, according to a least one of the preceding clauses, which contains one or more deuterated derivatives of L-DOPA as well as physiologically acceptable salts thereof.
7. The pharmaceutical composition, according to clause 6, further characterized in that the deuterated derivative of L-DOPA is selected from the group consisting of: 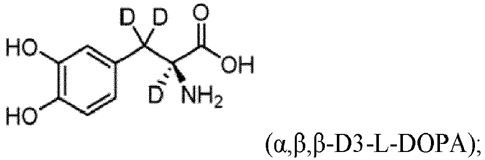 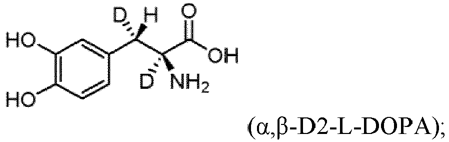 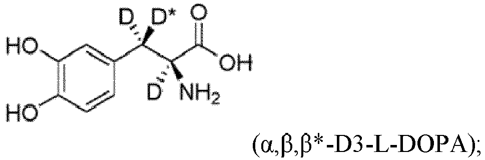 and 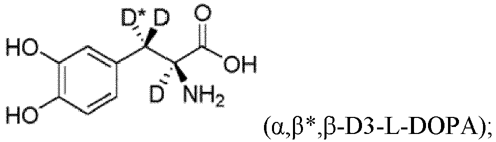 or physiological acceptable salts thereof, wherein each position designated as D or D* is enriched with deuterium.
8. The compound as recited in Clause 7 wherein each position designated as D has deuterium enrichment of no less than about 90%.
9. The compound as recited in Clause 7 wherein each position designated as D has deuterium enrichment of no less than about 96%.
10. The compound as recited in Clause 7 wherein each position designated as D has deuterium enrichment of no less than about 98%.
11. The compound as recited in Clause 7 wherein each position designated as D* has deuterium enrichment of about 80% to about 100%.
12. The compound as recited in Clause 7 wherein each position designated as D* has deuterium enrichment of about 85% to about 95%.
13. The compound as recited in Clause 7 wherein each position designated as D* has deuterium enrichment of about 88% to about 92%.
14. The compound as recited in Clause 7 wherein each position designated as D* has deuterium enrichment of about 90%.
15. The pharmaceutical composition according to at least one of the preceding clauses, further comprising at least one DOPA decarboxylase inhibitor.
16. The pharmaceutical composition, according to clause 15, wherein the DOPA decarboxylase inhibitor is selected from the group of (-)-L-α-hydrazino-3,4-dihydroxy-α-methylhydrocinnamic acid (carbidopa), D,L-serine 2-(2,3,4-trihydroxybenzyl) hydrazide (benserazide), L-serine-2-(2,3,4-trihydroxybenzyl) hydrazide, glycine-2-(2,3,4-trihydroxybenzyl) hydrazide or L-tyrosine-2-(2,3,4- trihydroxybenzyl) hydrazide, and physiological acceptable salts thereof.
17. The pharmaceutical composition according to at least one of the preceding clauses, wherein the dopamine agonist is apomorphine and wherein the concentration of apomorphine is between 2-30 mg/ml.
18. The pharmaceutical composition as recited in clause 17, wherein the concentration of apomorphine is about 5 mg/ml.
19. The pharmaceutical composition according to at least one of the preceding clauses, wherein the L-DOPA derivative is L-DOPA and wherein the concentration of L-DOPA is between 5-50 mg/ml.
20. The pharmaceutical composition as recited in clause 19, wherein the concentration of L-DOPA is between 10-15 mg/ml.
21. The pharmaceutical composition according to clause 15, wherein the DOPA decarboxylase inhibitor is carbidopa and wherein the concentration of carbidopa is between 0.5-10 mg/ml.
22. The pharmaceutical composition as recited in clause 21, wherein the concentration of carbidopa is about 2-3 mg/ml.
23. The pharmaceutical composition according to at least one of the preceding clauses, further comprising at least one cytoprotective compound.
24. The pharmaceutical composition as recited in clause 23, wherein the cytoprotective compound is selected from the group comprising N-acetylcysteine, cysteine and alpha lipoic acid.
25. A method for the preparation of the pharmaceutical composition according to at least one of the preceding clauses, comprising mixing at least one dopamine agonist and at least one L-DOPA derivative in a defined ratio to each other.
26. The method according to clause 13, further comprising a step of sterilizing the obtained mixture.
27. Use of the pharmaceutical composition according to at least one of the clauses 1 to 24, in addition to pharmaceutically acceptable adjuvants and additives, for the treatment of Parkinson's disease, restless leg syndrome, dystonia, for inhibiting prolactin secretion, for stimulating the release of growth hormones, for the treatment of neurological symptoms of chronic manganese intoxication, amyotrophic lateral scleroses, and multiple system atrophy.
28. Use of the pharmaceutical composition according to at least one of the clauses 1 to 24, in a non-oral application form selected from infusions, injections or an application via a gastric tube or an intestine tube.

From the foregoing description, one skilled in the art can easily ascertain the essential characteristics of this invention and without departing from the spirit and scope thereof, can make various changes and modifications of the invention to adapt it to various usages and conditions.

## Claims

1. A pharmaceutical composition comprising
i) a dopamine agonist; and
ii) one or more of a deuterated L-DOPA derivative, or a physiologically acceptable salt thereof,
in the form of a non-orally applicable, liquid preparation;
wherein the deuterated derivative of L-DOPA is: 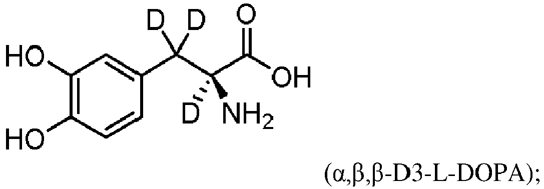 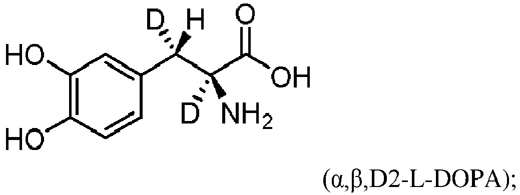 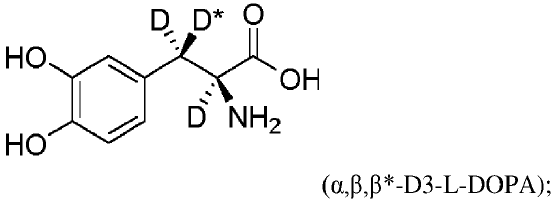 or 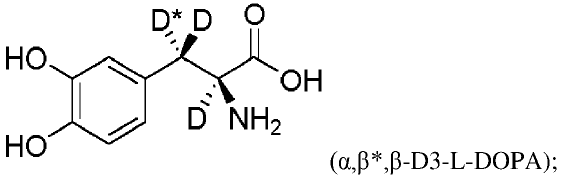 or a physiological acceptable salt thereof, or a combination thereof, wherein each position designated as D or D* is enriched with deuterium.

2. The pharmaceutical composition according to claim 1, further comprising a pharmaceutically acceptable adjuvant or additive which is a solvent, sugar, or pH regulator.

3. The pharmaceutical composition according to any one of the preceding claims, wherein the dopamine agonist is apomorphine, ropinirole, rotigotine, pramipexole, or piribedile.

4. The pharmaceutical composition according to Claim 1, wherein each position designated as D has deuterium enrichment of: no less than about 90%; no less than about 96%; or no less than about 98%; or wherein each position designated as D* has deuterium enrichment of: about 80% to about 100%; about 85% to about 95%; about 88% to about 92%; or about 90%.

5. The pharmaceutical composition according to claim 1, wherein the deuterated derivative of L-DOPA is α,β,β*-D3-L-DOPA having a deuterium enrichment of 90% in the βR position and which is a mixture of 10 mol% L-2-amino-2,3(S)-dideutero-3-(3,4-dihydroxyphenyl) propionic acid and 90 mol% L-2-amino-2,3,3-trideutero-3-(3,4-dihydroxyphenyl) propionic acid, wherein the deuterium enrichment of each of L-2-amino-2,3(S)-dideutero-3-(3,4-dihydroxyphenyl) propionic acid and L-2-amino-2,3,3-trideutero-3-(3,4-dihydroxyphenyl) propionic acid is >98% in all three positions.

6. The pharmaceutical composition according to any one of the preceding claims, further comprising at least one DOPA decarboxylase inhibitor,
preferably wherein the DOPA decarboxylase inhibitor is: (-)-L-α-hydrazino-3,4-dihydroxy-α-methylhydrocinnamic acid (carbidopa), D,L-serine 2-(2,3,4-trihydroxybenzyl) hydrazide (benserazide), L-serine-2-(2,3,4-trihydroxybenzyl) hydrazide, glycine-2-(2,3,4-trihydroxybenzyl) hydrazide or L-tyrosine-2-(2,3,4-trihydroxybenzyl) hydrazide, or a physiological acceptable salt thereof.

7. The pharmaceutical composition according to any one of the preceding claims, wherein the dopamine agonist is apomorphine and wherein the concentration of apomorphine is between 2-30 mg/ml, preferably wherein the concentration of apomorphine is about 5 mg/ml.

8. The pharmaceutical composition according to any preceding claim, wherein the concentration of deuterated L-DOPA derivative is between 10-15 mg/ml.

9. The pharmaceutical composition according to claim 6, wherein the DOPA decarboxylase inhibitor is carbidopa and wherein the concentration of carbidopa is between 0.5-10 mg/ml, and preferably wherein the concentration of carbidopa is about 2-3 mg/ml.

10. The pharmaceutical composition according to any one of the preceding claims, further comprising at least one cytoprotective compound, preferably wherein the cytoprotective compound is N-acetylcysteine, cysteine or alpha lipoic acid.

11. A method for preparing the pharmaceutical composition according to any one of the preceding claims, comprising mixing at least one dopamine agonist and at least one L-DOPA derivative in a defined ratio to each other.

12. The method according to claim 11, further comprising sterilizing the mixture.

13. The pharmaceutical composition according to any one of claims 1 to 10 for use in treating Parkinson's disease, treating restless leg syndrome, treating dystonia, inhibiting prolactin secretion, stimulating the release of growth hormones, treating neurological symptoms of chronic manganese intoxication, treating amyotrophic lateral scleroses, or treating multiple system atrophy.

14. Use of the pharmaceutical composition according to any one of claims 1 to 10, in a non-oral application form of an infusion, an injection, or a gastric tube, or an intestine tube.
